# EUROPEAN PATENT SPECIFICATION

(11) **EP 2 874 660 B1**
(45) Date of publication and mention of the grant of the patent: **23.01.2019**
(21) Application number: 13745508.5
(22) Date of filing: 18.07.2013
(51) Int. Cl.: A61K 45/06, A61P 27/10, A61K 9/00, A61K 31/137, A61K 31/167, A61K 31/4168, A61K 31/4174, A61K 31/4178, A61K 31/4402

(54) **OPHTHALMIC FORMULATION AND METHOD FOR AMELIORATING PRESBYOPIA**
OPHTHALMISCHE FORMULIERUNG UND VERFAHREN ZUR VERBESSERUNG VON PRESBYOPIE
FORMULATION OPHTALMIQUE ET PROCÉDÉ D'AMÉLIORATION DE LA PRESBYTIE

(30) Priority: 19.07.2012 US 201213553615
(43) Date of publication of application: 27.05.2015
(73) Proprietor: Vejarano Restrepo, Luis Felipe, Popayan (CO)
(72) Inventor: Vejarano Restrepo, Luis Felipe, Popayan (CO)
(74) Representative: Arth, Hans-Lothar
(86) International application number: PCT/US2013/051153
(87) International publication number: WO 2014/015183

(56) References cited:
- EP-A1- 1 938 839
- WO-A1-93/25199
- WO-A1-2010/125416
- WO-A2-2013/041967
- US-A1- 2009 156 606
- US-A1- 2011 152 274
- MARTIN X D ET AL: "Vasoconstrictive effect of topical timolol on human retinal arteries.", GRAEFE'S ARCHIVE FOR CLINICAL AND EXPERIMENTAL OPHTHALMOLOGY = ALBRECHT VON GRAEFES ARCHIV FÜR KLINISCHE UND EXPERIMENTELLE OPHTHALMOLOGIE 1989, vol. 227, no. 6, 1989, pages 526-530, XP008164927, ISSN: 0721-832X
- M D Eltze: "Affinity of the Miotic Drug, Dapiprazole, at alpha-1-Adrenoceptor Subtypes A, B and D", J. Pharm. Pharmacol., vol. 49, no. 30, 21 March 1997 (1997-03-21), pages 1091-1095, XP055126822,

## Description

### BACKGROUND OF THE INVENTION

### Field of the Invention

This application relates to a pharmaceutical preparation comprising pilocarpine or a pharmaceutically acceptable salt thereof, phenylephrine or a pharmaceutically acceptable salt thereof, and naphazoline or a pharmaceutically salt thereof, for ameliorating, reducing or treating presbyopia.

### Description of the Related Art

Presbyopia is an age-related reduction in visual acuity due to a decline in near focusing ability, commonly associated with blurred appearance of objects at nearby distances. Symptoms of presbyopia often become noticeable by around age 40 to around age 45. Presbyopia is typically associated with the reduced accommodative ability of the eye. For example, flexibility or elasticity of the crystalline lens and strength of the ciliary muscles often decrease with age. A decrease in the flexibility or elasticity of the crystalline lens or the strength of ciliary muscles can be associated with a decrease in the ability of the eye in adjusting the curvature of the crystalline lens to focus on objects at nearby distances, including objects at around a normal reading distance.

Common treatments of presbyopia include use of eye glasses, such as reading glasses typically worn for near distance vision, and bi-focals or multi-focals to provide both improved near and distance vision for patients who already use correction for distance vision. Corrective contact lenses can also be used to treat presbyopia, including bifocal, multi-focal or monovision contact lenses. Monovision contact lenses typically include a lens for distance vision in one eye, for example a dominant eye, and a lens for near distance vision in the other eye, for example a non-dominant eye. Surgical options are also available for treating presbyopia, including corrective eye surgery. For example, refractive eye surgery generally involves reshaping of the cornea. Refractive surgery can allow reshaping of the cornea in one eye while leaving the other eye untreated, for example correcting vision only in a non-dominant eye for improved near vision while allowing the dominant eye to maintain distance vision. Implantation of intraocular lenses (IOL) can be another surgical option in treating presbyopia, generally involving replacement of the natural lens with a synthetic one. However, eye glasses or corrective lenses may be cumbersome or provide inadequate treatment, while surgical correction can be invasive and are not without risks. Because these techniques merely compensate for the loss of accommodation by changing the way light enters the eye, patients would have to put on the glasses for near vision and remove the glasses for distance vision, or have only one eye corrected for near vision while the other eye remains uncorrected in order to maintain distance vision. Thus, a way to ameliorate or reduce the symptoms of presbyopia while allowing accommodation is needed.

US2011/0152274 discloses the combined use of pilocarpine and brimonidine for the treatment of presbyopia.

### SUMMARY OF THE INVENTION

In some embodiments, an ophthalmic formulation comprises an effective amount of pilocarpine, or a pharmaceutically acceptable salt thereof, phenylephrine or a pharmaceutically acceptable salt thereof, and naphazoline or a pharmaceutically acceptable salt thereof.

In some embodiments, said ophthalmic formulation is for use in a method of ameliorating, reducing or treating presbyopia which comprises administering an effective amount of the ophthalmic formulation to an eye of a patient.

### DETAILED DESCRIPTION OF SOME EMBODIMENTS

The embodiments described in this application relate to a pharmaceutical preparation as defined in the appended claims. In some embodiment, the pharmaceutical preparation described herein is useful for ameliorating, reducing and/or treating presbyopia or symptoms thereof. The pharmaceutical preparation may reduce or eliminate the symptoms of presbyopia while maintaining the accommodative function of the eyes, and allow patients suffering from presbyopia the ability to focus both far and near. In some embodiments, the pharmaceutical preparation can improve near vision of a patient suffering from presbyopia without affecting the distance vision. In some embodiments, the pharmaceutical preparation may be formulated into an ophthalmic formulation that can be applied to the eye of a patient suffering from conditions relating to presbyopia.

As used herein, the terms ameliorate, treat or treatment refer to a reduction in the severity of symptoms of an eye condition adversely affecting visual acuity. In some embodiments, an ophthalmic formulation as described herein is suitable for treating, or reducing the severity of the symptoms of, presbyopia. For example, an ophthalmic formulation as described herein may be suitable for the treatment of presbyopia by enabling the patient to visually focus on objects at a nearby distance, including objects at around a normal reading distance.

As used herein, the terms effective amount include quantities of one or more active ingredients described herein sufficient for the treatment of a condition of the eye adversely affecting visual acuity, including achieving temporary improvement in the symptoms of presbyopia. For example, effective amounts of an active ingredient when applied to one or both eyes of a patient may enable the patient to visually focus on objects at a nearby distance, including objects at a distance around a normal reading distance.

Some embodiments describe a pharmaceutical preparation comprising pilocarpine or a pharmaceutically acceptable salt thereof, phenylephrine or a pharmaceutically acceptable salt thereof, and naphazoline or a pharmaceutically salt thereof. In some embodiments, the pharmaceutical preparation is an ophthalmic formulation. In some embodiments, the ophthalmic formulation may further comprise one or more of ingredients selected from the group consisting of a an anti-histamine agent, a non-steroid anti-inflammatory drug, and an lubricant.

Pilocarpine is a direct acting parasympathomimetic agent that acts on M₃ muscarinic receptor. It can cause the ciliary muscle in the eye to contract and provide near focus. However, it may also cause contraction of the pupil or miosis.

Phenylephrine may dilate the pupil, and may reduce pupil contraction due to pilocarpine. In some embodiments, phenylephrine may contribute to the normal movement of pupil in any light situation, and/or reduces miosis. With proper concentrations of pilocarpine and phenylephrine, voluntary accommodation of the eyes can be maintained or restored while the presbyopia symptoms are ameliorated or treated.

In some embodiments of the ophthalmic formulation, pilocarpine or a pharmaceutically acceptable salt thereof, may be present in the amount of from about 0.1% to about 2.0%, from about 0.1% to about 1.9%, from about 0.2% to about 1.9%, including from about 0.3% to about 1.9%, from about 0.4% to about 1.9%, from about 0.2% to about 1.8%, from about 0.3% to about 1.7%, from about 0.1% to about 1.8%, from about 0.1% to about 1.5%, from about 0.1% to about 1.3%, from about 0.1% to about 1.2% from about 0.1% to about 0.9%, from about 0.1% to about 0.7%, from about 0.4% to about 1.6%, from about 0.5% to about 1.3%, or at about 0.4%, about 0.5%, about 0.6%, about 0.7%, about 0.8%, about 0.9%, about 1.0%, about 1.1%, about 1.2%, by weight.

In some embodiments of the ophthalmic formulation, the phenylephrine or a pharmaceutically acceptable salt thereof, may be present in the amount of from about 0.1% to about 7%, from about 0.1% to about 6%, from about 0.1% to about 5.5%, from about 0.1% to about 4.5%, from about 0.1% to about 3%, from about 0.2% to about 3.5%, from about 0.2% to about 4.5%, from about 0.2% to about 5.5%, from about 0.2% to about 6%, from about 0.3% to about 5%, from about 0.4% to about 4%, from about 0.5% to about 3%, from about 0.6% to about 3%, from about 0.6% to about 2.5%, from about 0.7% to about 2.5%, from about 0.7% to about 2.0%, or about 0.5%, about 1%, about 1.5%, about 2%, about 2.5%, about 3, about 3.5%, about 4%, about 5% by weight.

For example, an ophthalmic formulation may include by weight from about 0.001% to about 0.020%, including from about 0.002% to about 0.018%, including from about 0.004% to about 0.016%, including from about 0.006% to about 0.01%2, including from about 0.001% to about 0.015%, including from about 0.001% to about 0.012%, including from about 0.001% to about 0.011%, including from about 0.001% to about 0.010%, including from about 0.001% to about 0.009%, including from about 0.001% to about 0.008%, including from about 0.002% to about 0.008%, including from about 0.003% to about 0.009%, including about 0.002%, about 0.003%, about 0.004%, about 0.005%, about 0.006%, about 0.007%, about 0.008%, about 0.009%, about 0.010%, or about 0.012%, of naphazoline, or a pharmaceutically acceptable salt thereof.

In some embodiments, an ophthalmic formulation also optionally comprises one or more anti-histamine agents. A suitable anti-histamine agent or drug may be independently selected from pheniramine, chlorpheniramine, dexchlorpheniramine, dexbrompheniramine, deschlorpheniramine, triprolidine, brompheniramine, iodopheniramine, fluorpheniramine, or a pharmaceutically acceptable salt thereof. In some embodiments, the anti-histamine agent is pheniramine, or a pharmaceutically acceptable salt thereof. Pheniramine may serve to avoid conjunctival injection or congestion and reduce red eye. It also has a minimal effect on the ciliary muscle, and may improve accommodation. Pheniramine or a pharmaceutically acceptable salt thereof, may be present in an amount of from about 0.01% to about 0.20%, from about 0.05% to about 0.15%, from about 0.02% to about 0.10%, from about 0.03% to about 0.09%, from about 0.04% to about 0.08%, from about 0.02% to about 0.20%, from about 0.04% to about 0.15%, from about 0.04% to about 0.15%, at about 0.01%, about 0.02%, about 0.03%, about 0.04%, about 0.05%, about 0.06%, about 0.07%, about 0.08%, about 0.09%, or about 0.10% by weight in the ophthalmic formulation. Alternatively, an ophthalmic formulation may not comprise an anti-histamine agent.

In some embodiments, an ophthalmic formulation may further comprise a non-steroid anti-inflammatory drugs (NSAID). The NSAID may reduce or eliminate anterior segment inflammation. In some embodiments, a suitable NSAID is independently selected from the group consisting of nepafenac, meloxicam, diclofenac, bendazac, ketorolac, oxyphenbutazone, bromfenac, flurbiprofen, pranoprofen, surprofen, or indomethacin, and a pharmaceutically acceptable salt thereof. In some embodiments, an ophthalmic formulation comprises at least one of nepafenac or meloxicam. Alternatively, an ophthalmic formulation may not comprise a non-steroid anti-inflammatory drug.

In some embodiments, an ophthalmic formulation may be formulated to further comprise an effective amount of a NSAID nepafenac. For example, an ophthalmic formulation formulated to comprise nepafenac may include by weight from about 0.01% to about 0.10%, including from about 0.02% to about 0.09%, including from about 0.03% to about 0.08%, including from about 0.04% to about 0.07%, including from about 0.01% to about 0.09%, including from about 0.01% to about 0.08%, including from about 0.01% to about 0.07%, including from about 0.01% to about 0.06%, including from about 0.01% to about 0.05%, including from about 0.01% to about 0.04%, including from about 0.01% to about 0.03%, including from about 0.02% to about 0.08%, including from about 0.02% to about 0.07%, including from about 0.02% to about 0.06%, including from about 0.02% to about 0.05%, including about 0.02%, about 0.04%, about 0.03%, about 0.04%, or about 0.05% of nepafenac, or a pharmaceutically acceptable salt thereof.

Alternatively, the NSAID may be meloxicam. For example, the ophthalmic formulation may include by weight from about 0.001% to about 0.015%, including from about 0.001% to about 0.014%, including from about 0.001% to about 0.013, including from about 0.001% to about 0.014%, including from about 0.001% to about 0.014%, including from about 0.001% to about 0.012%, including from about 0.01% to about 0.009%, including from about 0.001% to about 0.008%, including from about 0.002% to about 0.007%, including from about 0.002% to about 0.006%, including from about 0.003% to about 0.012%, including from about 0.003% to about 0.01%, including from about 0.003% to about 0.009%, including from about 0.004% to about 0.012%, including about 0.003%, about 0.004%, about 0.005%, about 0.006%, about 0.007%, about 0.008%, about 0.009%, about 0.010%, or about 0.011% of meloxicam, or a pharmaceutically acceptable salt thereof.

An ophthalmic formulation, as described herein, may also further comprise a lubricant or lubricating agent. In some embodiments, the lubricant or lubricating agent may facilitate administration of the ophthalmic formulation. Suitable lubricants can be independently selected from polyethyleneglycol 400 or propyleneglycol. In some embodiments, the ophthalmic formulation comprises one or more suitable lubricants or lubricating agents. Alternatively, an ophthalmic formulation may not comprise a lubricant or lubricating agent.

In some embodiments, where polyethyleneglycol 400 is selected as the lubricant for the ophthalmic formulation, the ophthalmic formulation may comprise by weight of from about 0.01% to about 0.30%, including from about 0.02% to about 0.25%, 0.03% to about 0.20%, 0.04% to about 0.15%, 0.05% to about 0.15%, 0.05% to about 0.10%, 0.10% to about 0.30%, 0.10% to about 0.20%, 0.06% to about 0.20%, 0.05% to about 0.20%, including about 0.05%, about 0.10%, or about 0.15% of polyethyleneglycol 400 or a pharmaceutically acceptable salt thereof.

In some embodiments, the lubricant may be propyleneglycol. For example, an ophthalmic formulation may include by weight of from about 0.01% to about 0.20%, including from about 0.02% to about 0.10%, including from about 0.04% to about 0.09%, including from about 0.04% to about 0.08%, including from about 0.04% to about 0.06%, including from about 0.02% to about 0.10%, including from about 0.02% to about 0.12%, including from about 0.02% to about 0.14%, including from about 0.05% to about 0.20%, including from about 0.05% to about 0.15%, including about 0.01%, about 0.02%, about 0.03%, about 0.04%, about 0.05%, about 0.06%, about 0.07%, about 0.08%, about 0.09%, or about 0.10% of propyleneglycol or a pharmaceutically acceptable salt thereof.

In some embodiments, the ophthalmic formulation comprises one or more components to facilitate application of the ophthalmic formulation. For example, the ophthalmic formulation may also optionally comprise a pharmaceutically acceptable carrier, antibacterial agent, or a preservative. In some embodiments, a pharmaceutically acceptable carrier comprises purified water.

Also disclosed is a method of treating a condition of the eye adversely affecting near distance visual acuity of a patient, including ameliorating symptoms of presbyopia. A method of treating the symptoms of presbyopia may include applying to one or both eyes of a patient an ophthalmic formulation as described herein. The ophthalmic formulation may be applied to the eye of a patient as a liquid, a gel, a solution, a suspension, or any combination thereof. In some embodiments, the ophthalmic formulation is administered to the eye via an ocular route, including topical application to the conjunctiva.

The ophthalmic formulation comprises more components, each component may be administered sequentially or simultaneously to the one or both eyes of a patient for improved visual acuity, including for the treatment of the symptoms of presbyopia. For example, application of pilocarpine or a pharmaceutically acceptable salt thereof, phenylephrine or a pharmaceutically acceptable salt thereof, and naphazoline or a pharmaceutically salt thereof, may ameliorate symptoms of presbyopia while allowing voluntary accommodation.

In some embodiments, an ophthalmic formulation as described herein is applied to one or both eyes of a patient showing symptoms of presbyopia to improve the ability of the patient to focus on objects at a nearby distance, including objects at around a normal reading distance. In some embodiments, application of an ophthalmic formulation as described herein may sufficiently improve the near distance visual acuity of a patient independent of other methods of treatment. For example, administration of an ophthalmic formulation as described herein may enable a patient to focus on objects at a distance around a normal reading distance without use of corrective lenses or corrective eye surgery. Administration of the ophthalmic formulation may relieve symptoms for a patient in the early stages of presbyopia, including enabling near distance visual acuity without use of corrective lenses or glasses.

In some embodiments, an ophthalmic formulation as described herein is administered to a patient having symptoms of presbyopia, in addition to symptoms from myopia or hyperopia to facilitate near distance visual acuity such that the patient may not need to depend on the corrective treatment such as bifocals/multi-focals lenses or monovision contact lenses or to remove the glasses to read in myopes.

In some embodiments, an ophthalmic formulation as described herein is administered to one or both eyes of a patient to provide treatment for presbyopia as an alternative to corrective eye surgery. For example, an ophthalmic formulation as described herein may be administered to a patient having symptoms of presbyopia where the patient either prefers not to or cannot receive corrective eye surgery to treat presbyopia. The ophthalmic formulation as described herein may also be administered to a myopic or hyperopic patient (with or without astigmatism) having symptoms of presbyopia, but prefers to receive treatment only for the far vision defect. Alternatively, an ophthalmic formulation as described herein may be administered to a patient who continues to have symptoms of presbyopia after the patient has undergone corrective eye surgery for presbyopia. The ophthalmic formulation may be used in conjunction with corrective eye surgery for presbyopia to further reduce the symptoms of presbyopia. In some embodiments, the ophthalmic formulation as described herein is applied to one or both eyes of a patient to reduce a decline in near distance visual acuity after undergoing a corrective eye surgery for far vision at a younger age.

In some embodiments, administration of an ophthalmic formulation as described herein facilitates improvement in the symptoms of presbyopia for a patient who has reversed a previously received corrective eye surgery for presbyopia. For example, administration of the ophthalmic formulation may enable reestablishment of binocularity for a patient after a reversal or regression of a previously received monovision laser surgery for treating presbyopia.

In some embodiments, an ophthalmic formulation as described herein is administered to a patient to improve the ability of the patient to focus on nearby objects after the patient has undergone corrective surgery for treatment of ocular conditions other than presbyopia, including for example a corrective eye surgery for the treatment of a cataract.

In some embodiments, an ophthalmic formulation as described herein is used in conjunction with other treatments for eye conditions, including treatments for the symptoms of presbyopia. For example, an ophthalmic formulation as described herein may be administered to a patient in conjunction with use of mono-focal intraocular lenses, multi-focal intraocular lenses, or accommodative intraocular lenses to improve the near focusing ability of the patient.

In some embodiments, application of an ophthalmic formulation as described herein is suitable for reducing the symptoms of ocular hypertension. The ophthalmic formulation may be administered to an eye of a patient to improve symptoms of ocular hypertension through a reduction in intraocular pressure.

### EXAMPLES

A Prospective study was performed under a clinical protocol approved by the Clinic's Ethics Committee in Colombia. All subjects had signed an informed consent. A group of 20 presbyopia subjects with an average age of 49.65 (between 41 and 57 years old) participated in this study. Of the 20 presbyopia subjects, 9 were emmetropes, 5 were post-LASIK, and 6 were post-PresbV LASIK. The emmetropes are those who had perfect far vision but need glasses to correct the near vision. The post-LASIK subjects are those who received refractive surgery to correct their far visions before developing presbyopia. The post-PresbV LASIK subjects are those who previously received refractive surgery to correct presbyopia, and noticed a slight decrease in the near vision through time.

Each subject was given one drop of the ophthalmic formulation A in each eye. Various vision measurements were taken prior to the administration of the ophthalmic formulation A, and then 0.5 hour, 1 hour, 2 hours, 3 hours, 4 hours, 5 hours, 1 week and 1 month post administration in each eye and then binocularly. The vision measurements performed include uncorrected visual acuity in 20/20 format for distance and Jaeger for near (UCVA), best corrected visual acuity (BCVA), Refraction for far and near, pupil size, Schirmer Test, Endothelial Cell Count, Intra Ocular Pressure (IOP), Keratometry, and Anterior Chamber Depth (ACD).

The active ingredients of the ophthalmic formulation A include: pilocarpine 0.247% and Phenilephrine 0.78%. Additional ingredients include: Polyethyleneglycol as lubricant 0.09%, nepafenac 0.023%, and pheniramine 0.034%, and naphazoline 0.003%.

### UCVA Measurements

**Table 1: Binocular UCVA results for near vision in emmetropes, post-LASIK subjects, post-PresbV subjects, and average of all subjects (Jaeger format).**

| UCVA Near (Jaeger) | Emmetropes | Post-LASIK | Post-PresbV | Overall |
|---|---|---|---|---|
| Pre-eye drop | 4.44 | 2.16 | 3.0 | 3.44 |
| 0.5 hour post | 3.07 | 1.08 | 1.83 | 2.20 |
| 1 hour | 2.29 | 1.08 | 1.83 | 1.85 |
| 2 hours | 1.96 | 1.18 | 1.63 | 1.67 |
| 3 hours | 2.07 | 0.88 | 1.67 | 1.65 |
| 4 hours | 2.29 | 1.08 | 2.17 | 1.95 |
| 5 hours | 2.51 | 0.84 | 2.00 | 1.94 |
| 1 week | 3.29 | 1.52 | 1.97 | 2.45 |
| 1 month | 3.51 | 1.12 | 1.83 | 2.41 |

The binocular UCVA results for near vision shows that the ophthalmic formulation is effective in correcting the near vision in the presbyopia subjects, regardless of whether they had a perfect distance vision or previously had refractive surgeries to correct either the distance or near vision. The same results were also obtained for measurements performed on each eye separately, indicating that the ophthalmic formulation treatment is effective in treating both eyes, and is not a monovision treatment. The results indicate improvement of the near vision by 2-3 lines in each eye and binocularly. Surprisingly, with only 1 drop in each eye and administered once, the near vision improvement persists even at 1 week and at 1 month later, past the acting period of the ophthalmic formulation. Although the near vision improvement fades a little after the initial acting period, the potential long-term benefit of the ophthalmic formulation has been demonstrated with only 1 drop installation.

**Table 2: Binocular UCVA results for distance vision in emmetropes, post-LASIK subjects, post-PresbV subjects, and average of all subjects (20/20 format).**

| UCVA Far (20/20) | Emmetropes | Post-LASIK | Post-PresbV | Overall |
|---|---|---|---|---|
| Pre-eye drop | 20/23.61 | 20/24.5 | 20/27.5 | 20/25 |
| 0.5 hour post | 20/20.56 | 20/23 | 20/21.67 | 20/21.5 |
| 1 hour | 20/21.11 | 20/30 | 20/20 | 20/23 |
| 2 hours | 20/20.56 | 20/22 | 20/20.83 | 20/21 |
| 3 hours | 20/20 | 20/20 | 20/20 | 20/20 |
| 4 hours | 20/20 | 20/20 | 20/20 | 20/20 |
| 5 hours | 20/20 | 20/21 | 20/21.67 | 20/20.75 |
| 1 week | 20/20 | 20/21 | 20/20.83 | 20/20.5 |
| 1 month | 20/20 | 20/20 | 20/20.83 | 20/20.25 |

The binocular UCVA results for distance vision shows that the ophthalmic formulation does not adversely affect the visual acuity for the distance vision of the presbyopia subjects, regardless of whether they had a perfect distance vision or previously had refractive surgeries to correct either the distance or near vision. The same results were also obtained for measurements performed on each eye separately. Furthermore, the results also indicate improvement of the distance vision by between one and two lines binocularly and generally by one line in each eye. Thus, the results for binocular UCVA can be attributed to the improvements in both eyes. This also shows that, unlike other presbyopia eye drops, the ophthalmic formulation disclosed herein is effective in improving the near vision without compromising or adversely affecting the distance vision.

### Refraction Measurements

The refraction of the eye pre- and post-administration of 1 drop of ophthalmic formulation was measured. The changes in sphere and cylinder are calculated from the measurements and displayed in Table 3. The cylinder was not changed by the ophthalmic formulation, while a mild change in the sphere was observed. The change in the Sphere is most noticeable at 1 hour after the administration of the formulation, but the change is only a half of diopter. Since the cylinder does not change and only mild change is observed in the sphere, it only caused a mild myopic shift, and thus no adverse effect on the distance vision. In comparison, other presbyopia drops cause a large myopic shift to improve the near vision, but that also decrease the distance vision.

**Table 3: Refractive change following the administration of 1 drop of ophthalmic formulation (measure in Diopters).**

| | Pre | 0.5 h | 1 h | 2 h | 3 h | 4 h | 5 h | 1 week | 1 mo |
|---|---|---|---|---|---|---|---|---|---|
| Sphere | 0 | -0.39 | -0.51 | -0.36 | -0.23 | -0.11 | -0.10 | -0.07 | -0.01 |
| Cylinde r | 0 | 0.02 | -0.04 | -0.01 | -0.01 | 0.01 | -0.03 | -0.01 | -0.01 |

### Capacity of Accommodation

The optical quality of the eye was measured using an OQAS HD Analyzer prior to the administration of the ophthalmic formulation and 2 hours after administration of 1 drop in each eye. The results show that the objective scatter index (OSI) of the eye is not affected by the ophthalmic formulation disclosed herein, the accommodative range improved about 0.75 diopters after the administration of the ophthalmic formulation. An aberrometer (iTrace) was also used to measure objectively the capacity of accommodation for distance and near visions, and the results agree with that of OQAS measurement.

### Pupil Size

The change in the pupil diameter was also found to be minimal when measured by OQAS, iTrace, Pentacam, and Autorefractor. This further confirms that the ophthalmic formulation disclosed herein does not affect the pupil diameter too much.

**Table 4: Pupil diameter changes after administration of the ophthalmic formulation. (Measure in mm)**

| Pupil diameter | Pre | 0.5 h | 1 h | 2 h | 3 h | 4h | 5h | 1 wk | 1 mo |
|---|---|---|---|---|---|---|---|---|---|
| Autorefractor | 4.86 | 4.64 | 4.78 | 4.74 | 4.55 | 4.42 | 4.42 | 4.72 | 4.79 |
| Pentacam | 3.12 | 3.67 | 3.77 | 3.47 | 3.21 | 3.13 | 3.07 | 3.14 | 3.09 |

### Anterior Chamber Depth (ACD)

The ACD was measured by Pentacam, and the results are shown in Table 5. The anterior chamber depth shallow occurred slightly, but not enough to result in problems. This is different from other presbyopia formulations because other formulations would shallow the ACD to achieve improved near vision.

**Table 5: ACD of the eyes at various times after administration of the ophthalmic formulation. (Measure in mm)**

| | Pre | 0.5 h | 1 h | 2 h | 3 h | 4 h | 5 h | 1 wk | 1 mo |
|---|---|---|---|---|---|---|---|---|---|
| ACD | 3.21 | 3.16 | 3.16 | 3.18 | 3.16 | 3.16 | 3.13 | 3.19 | 3.21 |

### Intra Ocular Pressure (IOP)

The IOP was measured by Goldmann and Pascal Tonometers, and the results are shown in Table 6. The IOP is important for evaluating the safety of the ophthalmic formulation, as high IOP can be dangerous to the optic nerve and can cause irreversible damages with time. The results show that the ophthalmic formulation does not increase the IOP, and thus is safe.

**Table 6: IOP of the eyes at various times before and after administration of the ophthalmic formulation. (Measure in mm of Hg)**

| IOP | Pre | 0.5 h | 1 h | 2 h | 3 h | 4 h | 5 h | 1 wk | 1 mo |
|---|---|---|---|---|---|---|---|---|---|
| Pascal | 17.34 | 17.31 | 17.14 | 17.46 | 16.54 | 16.43 | 15.61 | 17.09 | 17.06 |
| Goldmann | 14.28 | 14.23 | 13.90 | 14.18 | 13.25 | 13.03 | 12.55 | 13.68 | 14.08 |

### Tear Quantity and Quality

The Schirmer test measures the quantity of lacrimal tear. The results show that the ophthalmic formulation does not have an adverse effect on the lacrimal film, thus less likely to cause dry eye problems. The quality of the tear film post-administration of the ophthalmic formulation was also measured using OQAS. The results show that the ophthalmic formulation has no adverse effect on the quality of the tear film as well in the long term use.

### Keratometry

The corneal curvature at its flat and steep meridian was measured pre- and post-administration of 1 drop of ophthalmic formulation at various intervals. The results show that the corneal curvature is the most steep at half hour and 1 hour after administration when the ophthalmic formulation has the most effect and when the major accommodation occurs.

### Endothelial Cell Count

Endothelial cells are found on the cornea, and the corneal endothelial cell count can indicate the health of the cornea. The endothelial cell counts of the subjects were collected as a part of the ophthalmic formulation safety study. The results show that the endothelial cell count does not decrease after administration of the ophthalmic formulation, which indicates the ophthalmic formulation has no adverse effect on the cornea and is safe for long term use.

## Claims

1. An ophthalmic formulation comprising an effective amount of pilocarpine or a pharmaceutically acceptable salt thereof, phenylephrine or a pharmaceutically acceptable salt thereof, and naphazoline or a pharmaceutically acceptable salt thereof.

2. The ophthalmic formulation of Claim 1, wherein the phenylephrine is present in an amount of 0.5 % to 3 % by weight.

3. The ophthalmic formulation of Claim 2, wherein the phenylephrine is present in an amount of 0.7 % to 2.2 % by weight.

4. The ophthalmic formulation according to any one of Claims 1-3, wherein the pilocarpine is present in an amount of 0.1 % to 0.7 % by weight.

5. The ophthalmic formulation according to any one of Claims 1-4 further comprising an anti-histamine selected from the group consisting of pheniramine, chlorpheniramine, dexchlorpheniramine, dexbrompheniramine, deschlorpheniramine, triprolidine, brompheniramine, iodopheniramine, fluorpheniramine, and a pharmaceutically acceptable salt thereof.

6. The ophthalmic formulation of Claim 5, wherein the anti-histamine is pheniramine or a pharmaceutically acceptable salt thereof.

7. The ophthalmic formulation of Claim 6, wherein the pheniramine is present in an amount of 0.03% to 0.09% by weight.

8. The ophthalmic formulation according to any one of Claims 5-7, further comprising a non-steroidal anti-inflammatory drug selected from the group consisting of nepafenac, meloxicam, diclofenac, bendazac, ketorolac, oxyphenbutazone, bromfenac, flurbiprofen, pranoprofen, surprofen, indomethacin, and a pharmaceutically acceptable salt thereof.

9. The ophthalmic formulation of Claim 8, wherein the non-steroidal anti-inflammatory drug is selected from the group consisting of nepafenac and meloxicam.

10. The ophthalmic formulation according to any one of Claims 1-9 for use in ameliorating, reducing or treating presbyopia.

11. The ophthalmic formulation for use according to Claim 10, wherein the phenylephrine is present in an amount of 0.5 % to 3 % by weight and the pilocarpine is present in an amount of 0.1 % to 0.7 % by weight.

## Patentansprüche

1. Eine ophthalmische Formulierung umfassend eine wirksame Menge an Pilocarpin oder ein pharmazeutisch verträgliches Salz davon, Phenylephrin oder ein pharmazeutisch verträgliches Salz davon und Naphazolin oder ein pharmazeutisch verträgliches Salz davon.

2. Die ophthalmische Formulierung nach Anspruch 1, wobei das Phenylephrin in einer Menge von 0,5 Gew.-% bis 3 Gew.-% enthalten ist.

3. Die ophthalmische Formulierung nach Anspruch 2, wobei das Phenylephrin in einer Menge von 0,7 Gew.-% bis 2,2 Gew.-% enthalten ist.

4. Die ophthalmische Formulierung nach einem der Ansprüche 1-3, wobei das Pilocarpin in einer Menge von 0,1 Gew.-% bis 0,7 Gew.-% enthalten ist.

5. Die ophthalmische Formulierung nach einem der Ansprüche 1-4, weiter umfassend ein Antihistaminikum ausgewählt aus der Gruppe bestehend aus Pheniramin, Chlorpheniramin, Dexchlorpheniramin, Dexbrompheniramin, Deschlorpheniramin, Triprolidin, Brompheniramin, lodpheniramin, Fluorpheniramin und ein pharmazeutisch verträgliches Salz davon.

6. Die ophthalmische Formulierung nach Anspruch 5, wobei das Antihistaminikum Pheniramin oder ein pharmazeutisch verträgliches Salz davon ist.

7. Die ophthalmische Formulierung nach Anspruch 6, wobei das Pheniramin in einer Menge von 0,03 Gew.-% bis 0,09 Gew.-% enthalten ist.

8. Die ophthalmische Formulierung nach einem der Ansprüche 5-7, weiter umfassend einen nichtsteroidalen antiinflammatorischen Wirkstoff ausgewählt aus der Gruppe bestehend aus Nepafenac, Meloxicam, Diclofenac, Bendazac, Keterolac, Oxyphenbutazon, Bromfenac, Flurbiprofen, Pranoprofen, Surpofen, Indometacin und ein pharmazeutisch verträgliches Salz davon.

9. Die ophthalmische Formulierung nach Anspruch 8, wobei der nichtsteroidale antiinflammatorische Wirkstoff aus der Gruppe bestehend aus Nepafenac und Meloxicam ausgewählt ist.

10. Die ophthalmische Formulierung nach einem der Ansprüche 1-9, zur Verwendung in der Linderung, Verringerung oder Behandlung von Presbyopie.

11. Die ophthalmische Formulierung zur Verwendung nach Anspruch 10, wobei das Phenylephrin in einer Menge von 0,5 Gew.-% bis 3 Gew.-% enthalten ist und das Pilocarpin in einer Menge von 0,1 Gew.-% bis 0,7 Gew.-% enthalten ist.

## Revendications

1. Une formulation ophtalmique comprenant une quantité efficace de pilocarpine ou un sel de celle-ci de formule acceptable dans le domaine pharmaceutique, de la phényléphrine ou un sel de celle-ci de formule acceptable dans le domaine pharmaceutique, ainsi que de la naphazoline ou un sel de celle-ci de formule acceptable dans le domaine pharmaceutique.

2. La formulation ophtalmique de la revendication 1, où la phényléphrine est présente en une quantité de 0,5 % à 3 % du poids.

3. La formulation ophtalmique de la revendication 2, où la phényléphrine est présente en une quantité de 0,7 % à 2,2 % du poids.

4. La formulation ophtalmique selon l'une des revendications 1 à 3, où la pilocarpine est présente en une quantité de 0,1 % à 0,7 % du poids.

5. La formulation ophtalmique selon l'une des revendications 1 à 4, comprenant également un anti-histaminique sélectionné parmi le groupe incluant la phéniramine, chlorphéniramine, dexchlorphéniramine, dexbromphéniramine, dexchlorphéniramine, triprolidine, bromphéniramine, iodophéniramine, fluorphéniramine et un sel de formule acceptable dans le domaine pharmaceutique.

6. La formulation ophtalmique de la revendication 5, où l'anti-histaminique est la phéniramine ou un sel de celle-ci de formule acceptable dans le domaine pharmaceutique.

7. La formulation ophtalmique de la revendication 6, où la phéniramine est présente en une quantité de 0,03 % à 0,09 % du poids.

8. La formulation ophtalmique selon l'une des revendications 5 à 7, comprenant également un anti-inflammatoire non stéroïdien sélectionné parmi le groupe incluant le népafénac, méloxicam, diclofénac, bendazac, kétorolac, oxyphenbutazone, bromfénac, flurbiprofène, pranoprofène, surprofène, indométacine et un seul de formule acceptable dans le domaine pharmaceutique.

9. La formulation ophtalmique de la revendication 8, où l'anti-inflammatoire non stéroïdien est sélectionné parmi le groupe comprenant le népafénac et le méloxicam.

10. La formulation ophtalmique selon l'une des revendications 1 à 9 pour une utilisation destinée à améliorer, traiter ou réduire la presbytie.

11. La formulation ophtalmique pour une utilisation selon la revendication 10, où la phényléphrine est présente en une quantité de 0,5 % à 3 % du poids total et la pilocarpine est présente en une quantité de 0,1 % à 0,7 % du poids total.
